(19) **Europäisches Patentamt**
**European Patent Office**
**Office européen des brevets**

(11) **EP 4 197 504 B1**

(12) **EUROPEAN PATENT SPECIFICATION**

(45) Date of publication and mention
of the grant of the patent:
**03.01.2024 Bulletin 2024/01**

(21) Application number: **21214275.6**

(22) Date of filing: **14.12.2021**

(51) International Patent Classification (IPC):
**A61F 13/15** (2006.01)   **A61F 13/42** (2006.01)
**A61F 13/505** (2006.01)   **A61F 13/49** (2006.01)

(52) Cooperative Patent Classification (CPC):
**A61F 13/42; A61F 13/15252; A61F 13/15268;**
**A61F 13/49003; A61F 13/505;** A61F 2013/15276;
A61F 2013/5055

(54) **RE-USABLE SHELL AND DISPOSABLE INSERT WITH WETNESS INDICATOR**

WIEDERVERWENDBARE SCHALE UND WEGWERFEINSATZ MIT NÄSSEANZEIGER

COQUE RÉUTILISABLE ET INSERT JETABLE AVEC INDICATEUR D'HUMIDITÉ

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**21.06.2023 Bulletin 2023/25**

(73) Proprietors:
• **Ontex BV**
  **9255 Buggenhout (BE)**
• **Ontex Group NV**
  **9320 Erembodegem (BE)**

(72) Inventors:
• **COBBAERT, Dries**
  **1770 Liedekerke (BE)**

• **WEBER, Ainas**
  **53474 Bad Neuenahr-Ahrweiler (DE)**
• **Idelson, Alissa**
  **53359 Rheinbach (DE)**
• **Lambertz, Christina**
  **56566 Neuwied (DE)**

(74) Representative: **Macchetta, Andrea**
**Ontex BV**
**Korte Keppestraat 21**
**9320 Erembodegem-Aalst (BE)**

(56) References cited:
**EP-A1- 3 213 727        EP-A1- 3 824 856**
**US-A1- 2014 005 621**

## Description

## TECHNICAL FIELD

The disclosure relates to absorbent articles such as disposable absorbent articles, preferably selected from the group consisting of diapers (whether for baby or adults), pants (whether for baby or adults), briefs, and combinations thereof generally of the hybrid type i.e. a re-usable and/or washable outer shell and a disposable (generally single-use) absorbent insert cooperable with said outer shell.

## BACKGROUND

Hybrid-type articles, comprising a re-usable outer shell and disposable absorbent insert, are becoming more and more sought after in the market in view of their sustainable benefits and reduced waste production.
Different designs are available for such products such as described in EP 3 842 017 A1 wherein the outer shell is generally a pant-type component that may comprise elastic components therein and the disposable absorbent insert comprises liquid permeable topsheet, liquid impermeable backsheet and absorbent core sandwiched therebetween and is generally re-fastenably joinable to a crotch region of the outer shell.
Other examples are described in EP 3 659 563 A1 where the disposable insert is re-fastenably joined to the outer shell and the outer shell is in the form of a re-fastenable diaper pant; and EP 3 895 673 A1 wherein the disposable inserts are biodegradable and/or compostable.
Thus, although there have already been significant advancements in this field, there still remains a need to further improve hybrid-type articles so as to provide improved convenience of use such as correct location of the insert onto the outer shell (particularly when a specific front/back orientation is desirable) and/or indication of liquid saturation to provide a warning to the caregiver that the insert should be replaced with a new one.

## SUMMARY

In a first aspect, the disclosure relates to a disposable absorbent insert according to Claim 1 and Claims dependent therefrom.
In a second aspect, the disclosure relates to a re-usable outer shell comprising: a front and back waist region having uppermost and lowermost edges; and a crotch region interposed between the front and back waist region, wherein the uppermost edges of the waist region form a waist opening and the lowermost edges of the waist region together with lateral extremities of the crotch region form two oppositely disposed leg openings; and wherein at least the body-facing surface of the crotch region is adapted for receiving an absorbent insert according to any of the preceding claims; and wherein at least a portion of said crotch region corresponding with the wetness indicator of the insert is translucent and/or transparent such that said wetness indicator is viewable from a garment facing side of said re-usable outer shell or comprises an optical sensor adapted to automatically detect a colour change of said wetness indicator and send a corresponding signal to an application device.
In a further aspect, the disclosure relates to an absorbent assembly comprising an insert and a re-usable outer shell, preferably further comprising a detection device comprising an optical sensor.
In a further aspect, the disclosure relates to a kit of parts comprising a plurality of inserts and a re-usable outer shell, preferably wherein the plurality of inserts (1) differ in at least one of: size, absorbency, biodegradable and/or compostable content, shape, and number of components contained therein. More preferably, further comprising a detection device comprising an optical sensor.

## BRIEF DESCRIPTION OF THE FIGURES

Fig. 1A Is an illustration, top planar view (topsheet-side), of an insert according to an embodiment herein.

Fig. 1B Is an illustration, bottom planar view (backsheet-side), of an insert according to an embodiment herein.

Fig. 2 Is an illustration, cross-section view, of an insert according to an embodiment herein.

Fig. 3 Is an illustration, top planar view (body-facing side), of a re-usable outer shell according to an embodiment herein.

Fig. 4 Is an illustration, top planar view (body-facing side), of a re-usable outer shell according to an embodiment herein.

Fig. 5 Illustrates an exemplary process for the production of high-AUL Bio-SAP.

## DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.
As used herein, the following terms have the following meanings:
"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-20% or less, preferably +/-10% or less, more preferably +/-5% or less, even more preferably +/-1 % or less, and still more preferably +/-0.1 % or less of and from the specified value, in so far such variations are appropriate to perform in the disclosed disclosure. However, it is to be understood that the value to which the modifier "about" or "substantially" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" or "%wt" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

The terms "nonwoven", "nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The term "disposable" refers to absorbent articles and/or inserts that generally are not intended to be laundered or otherwise restored or reused as absorbent articles, i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a unitary structure with other elements or as a separate element joined to another element.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

The term "joined" refers to configurations whereby an element is directly secured to another element by attaching the element directly to the other element, and configurations whereby an element is indirectly secured to another element by attaching the element to intermediate member(s) which in turn are attached to the other element.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension of the article and when combined with the term "substantially" includes directions within ±45° of the longitudinal direction.

Embodiments of the articles according to the disclosure will now be described. It is understood that technical features described in one or more embodiments maybe combined with one or more other embodiments without departing from the intention of the disclosure and without generalization therefrom, especially when such combinations are explicitly or implicitly inferred.

THE OUTER SHELL

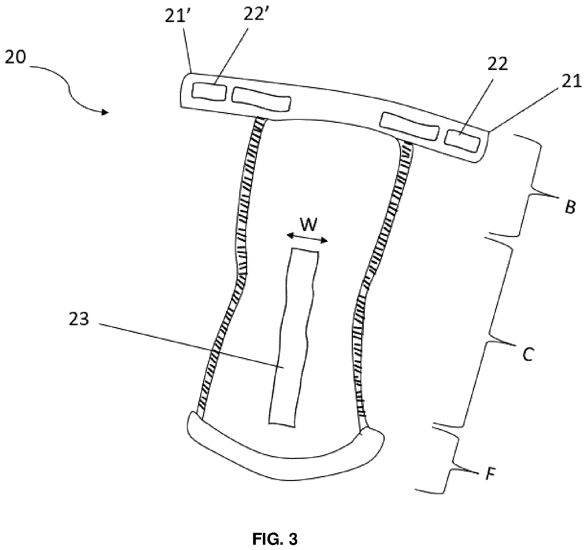

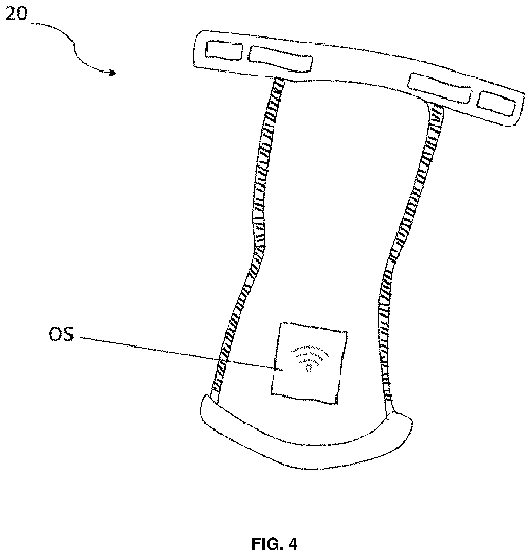

As exemplified in Figs. 3 and 4, re-usable outer shells (20) herein preferably comprise: a front (F) and back (B) waist region having uppermost and lowermost edges; and a crotch region (C) interposed between the front and back waist region (F, B), wherein the uppermost edges of the waist region form a waist opening and the lowermost edges of the waist region together with lateral extremities of the crotch region form two oppositely disposed leg openings; and wherein at least the body-facing surface of the crotch region (C) is adapted for receiving an absorbent insert (1) as described herein; and wherein at least a portion of said crotch region (C) corresponding with the wetness indicator (10) of the insert (1) is translucent such that said wetness indicator (10) is viewable from a garment facing side of said re-usable outer shell (20) or comprises an optical sensor (OS) adapted to automatically detect a colour change of said wetness indicator (10) and send a corresponding signal to an application device. Advantageously this not only allows to provide effective saturation indication when using a hybrid-article but further allows for at the same time providing indicia to correctly position the insert in the right front/back relationship that may be particularly useful when designing inserts that have a non-uniform absorption profile (e.g. varying basis weight of absorbent material for optimised absorption and limited over-specked or wasted material which then require the correct front/back orientation on placement). In the latter case the insert may be correctly placed when the wetness indicator is fully/entirely viewable through the outer shell (when viewed on the garment side thereof). This may be verified either directly, i.e. visually, by a caregiver viewing the wetness indicator indicia or indirectly, by automatic identification by the sensor that may then provide a warning via an application device to a caregiver accordingly.

The re-usable outer shell (20) may comprise re-fastenable or permanent side seams joining the front and back waist regions, preferably in the form of a pant; or the back waist region (B) comprises transversely extending side panels (21, 21') each comprising a fastener (22, 22') for coupling to a landing zone positioned at a garment facing side of the front waist region (F), preferably in the form of a diaper.

In an embodiment the crotch region (C) of the re-usable outer shell (20) comprises a window (23) for viewing said wetness indicator (10) and/or optical sensor (OS) from a garment-facing side of said shell (20). The crotch region (C) is generally adapted for receiving an absorbent insert (1) as described herein and wherein at least a portion and/or area of said crotch region comprises said window (23) that substantially corresponds with the position of the wetness indicator (10), when the insert (1) is joined to the shell (20). Advantageously this allows for visual inspection of saturation and/or correct positioning directly by a subject on use.

The window herein may be in the form of a cut-out or opening, or may comprise a translucent and/or transparent material such as a film or other suitable synthetic covering.

In an embodiment, the optical sensor (OS) is comprised by a detection device further comprising one or more light sources, preferably a plurality of light sources having at least a first colour and a second colour, wherein the first colour is blue and the second colour is red, preferably said light sources consisting of LEDs, more preferably said light sources being positioned at a distance from each other and from the optical sensor; even more preferably the detection device further comprising a battery and a transmitter and being detachably connected to said outer shell.

Optical sensors and devices suitable herein may be as described in more detail in co-pending application EP 3 888 606 A1.

In a preferred embodiment, the sensor and/or detection device is positioned on a body-facing surface of the re-usable shell with the optical sensor facing upwards towards a body-facing side such to come into, preferably direct, contact with the backsheet of the insert comprising the wetness indicator. Alternatively, the sensor and/or detection device may be positioned on a garment-facing surface of the re-usable shell at a position corresponding to the window (23) so that the optical sensor is in direct or indirect, preferably indirect, contact with the backsheet (3) of the insert (1) comprising the wetness indicator (10) via the window (23) described herein.

THE INSERT

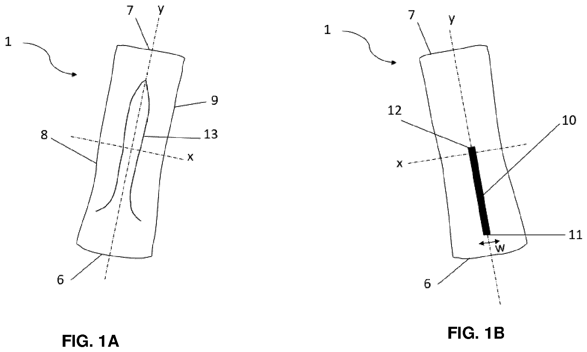

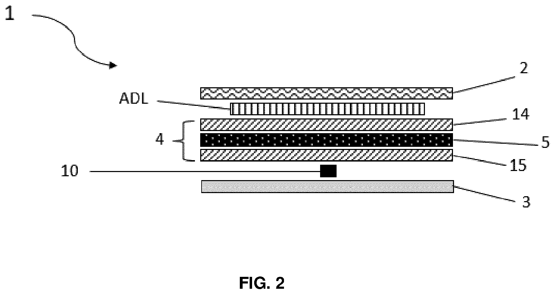

As exemplified in Figs. 1A, 1B, and 2, inserts (1) herein typically comprise a liquid permeable topsheet (2), a liquid impermeable backsheet (3); and an absorbent core (4) comprising absorbent material (5) therein and being sandwiched between said topsheet (2) and backsheet (3).

inserts herein may further comprise an acquisition distribution layer (ADL) positioned between the topsheet and the absorbent core, preferably between the topsheet and the top core wrap layer. The acquisition distribution layer may have a basis weight of from 15 gsm to 55 gsm, preferably from 18 gsm to 50 gsm, even more preferably from 19 gsm to 45 gsm. Preferably the acquisition distribution layer is selected from a carded air-through bonded nonwoven, a carded thermobonded calendered nonwoven, a spunbond nonwoven, and combinations thereof.

Generally, the insert comprises a perimeter formed by first and second transverse edges (6, 7) and first and second longitudinal edges (8, 9) connecting the first and second transverse edges (6, 7); a longitudinal centerline extending (y) substantially parallel to said first and second longitudinal edges (8, 9) and interposed therebetween such to divide said insert (1) into a first longitudinal half between said longitudinal centerline (y) and said first longitudinal edge (8) and a second longitudinal half between said longitudinal centerline (y) and said second longitudinal edge (9); and a transverse centerline (x) extending substantially parallel to said first and second transverse edges (6, 7) and interposed therebetween such to divide said insert (1) into a first transverse half between said transverse centerline (x) and said first transverse edge (6) and a second transverse half between said transverse centerline (x) and said second transverse edge (7).

Inserts (1) herein preferably comprises a wetness indicator (10) that is viewable from a garment-facing side of the backsheet (3) and the insert (1) is joinable to a re-usable outer shell (20) preferably such that the wetness indicator (10) is viewable from a garment-facing side of the re-usable outer shell (20).

Inserts herein preferably comprise a pair of barrier cuffs extending along the first and second longitudinal edges (8, 9) and arranged to provide lateral barriers preventing exudate leakage. Typically, the barrier cuffs comprising a hydrophobic nonwoven and one or more elastics at an apex position thereof such to form standing gathers when the insert is extended for placement within the re-usable outer shell.

Inserts herein are free of transversely extending side panels such as elastic side panels.

Backsheets for use herein are as commonly known in the art and may be breathable and/or comprise a laminate of a film, preferably polyethylene (PE) based or bio-PE based, and a nonwoven layer with the nonwoven layer being positioned on the outermost surface at a garment facing side thereof.

The inserts herein may be substantially rectangular in shape but may equally be shaped such as hourglass and/or substantially T-shaped. Other anatomical shapes are further contemplated herein and suitable in the present disclosure.

In an embodiment, the absorbent material comprises, preferably consists of, superabsorbent particles and/or cellulose fibers.

The absorbent cores herein may comprise at least 60%wt of superabsorbent particles, in particular at least 70%wt, preferably at least 80%wt, preferably at least 90%wt, by total weight of the core.

In an embodiment, the superabsorbent particles comprise a blend of superabsorbent particles comprising a first superabsorbent particles (SAP1) and a second superabsorbent particles (SAP2), wherein the first superabsorbent particles (SAP1) have an AUL that is greater than the AUL of the second superabsorbent particles (SAP2), and wherein the first superabsorbent particles (SAP1) have an AUL of greater than 15 g/g, according to the test method herein, preferably wherein the first superabsorbent particles (SAP1) have a particle size distribution that is greater than that of the second superabsorbent particles (SAP2). Preferably wherein at least the second superabsorbent particles (SAP2) comprises, preferably consists of, bio-based superabsorbent composite polymer particles comprising a synthetic hydrophobic polymer and a natural biopolymer, more preferably composed of a composite polymer comprising styrene maleic acid copolymer and a biopolymer of animal or vegetal origin; or non-composite polymer particles selected from polyacrylic acid, sodium salt, crosslinked, partly neutralized superabsorbent particles.

Preferably the second superabsorbent particles (SAP2) comprises, preferably consists of, Low-AUL Bio-SAP. "Low-AUL Bio-SAPs" means that they generally have AUL (as measured according to the test method herein) of less than 20 g/g, typically less than 15 g/g, and typically belong to the following classes: (a) materials consisting only of crosslinked biopolymers; (b) materials consisting only of crosslinked synthetic polymers; (c) composite materials consisting of synthetic polymers and biopolymers in certain variations: in inter crosslinking type connection with or without chemical agents; graft type; intercomplexate type and interpenetrate type. Copolymers such as styrene maleic acid are typically used in copolymerization processes for achieving Low-AUL Bio-SAP. Other exemplary sources include alpha-1,3 glucan, starch, starch and/or sodium salts, sugar and derivatives. Exemplary commercially available Low-AUL Bio-SAPs for use herein include: SAPs derived from charged-modified starches as sold from TETHIS 5237 Capital Blvd.; Raleigh, NC 27616, USA and further exemplified in US20200054782 A1 herein incorporated by reference; SAPs comprising a 100% acrylic acid co-acrylamide with little to no cross link shell, and the like all generally having AUL of less than 20 g/g according to the test method herein.

Preferably, the first superabsorbent particles (SAP1) are free of Low-AUL Bio-SAP. Advantageously this limits rewet drawbacks as described herein.

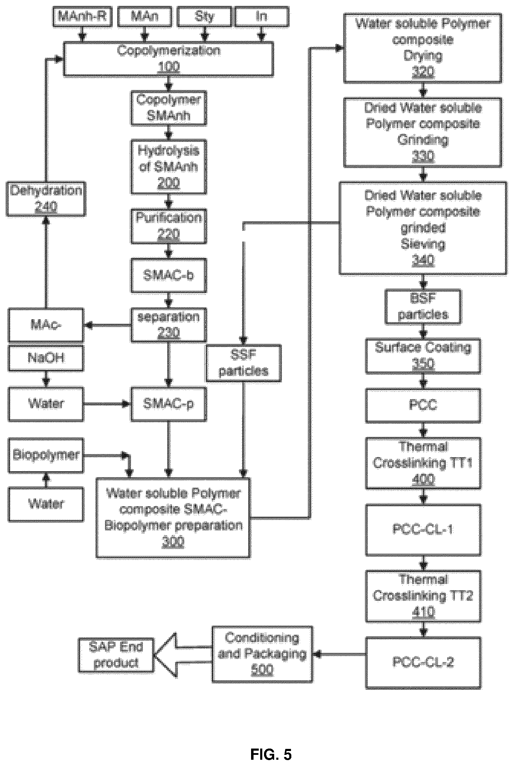

In an embodiment, the SAP1 is a High-AUL Bio-SAP "composite polymer" (i.e. a biodegradable superabsorbent composite polymer having an AUL of greater than 15 g/g, preferably greater than 20 g/g, as will be described in more detail herein below). The composite polymer may comprise a synthetic hydrophobic polymer and a natural biopolymer. More specifically, the composite polymer may comprise styrene maleic acid copolymer and a biopolymer of animal or vegetal origin in conformity with the technological flow chart presented in Fig. 5. Styrene maleic acid copolymer is preferably in salt form, more preferably in the form of monovalent cation salt. Alternatively, the High-AUL Bio-SAP, although less preferred, may comprise "non-composite polymers" and rather be selected from certified biomass superabsorbent polymers having AUL of greater than 15 g/g, preferably greater than 20 g/g, and typically certified by REDcert2 (https://www.redcert.org/images/SP_RC%C2%B2_Biomass-balanced_products_V1.0.pdf ). An example may be a polyacrylic acid, sodium salt, crosslinked, partly neutralized SAP from up to 100% allocated biomass feedstock such as commercially available HySorb® B 6600MB manufactured and sold by BASF SE, Ludwigshafen, Germany.

In an embodiment of the High-AUL Bio-SAP "composite polymer", the styrene moiety in the synthetic polymer can be replaced by other hydrophobic moieties. Exemplary synthetic polymers are: poly(maleic anhydride-co- methyl vinyl ether) (Gantrez), poly(vinyl chloride-co-maleic acid) and poly[(maleic anhydride)- alt-(vinyl acetate).

The term "composite" as herein used refers to a polymeric substance that a) is formed from at least two polymers with different macromolecular chemical structure; and b) the resulting composite is a unique entity that does not separate spontaneously to its components during application. It is understood that the term "composite" may include other substances such as drugs, stimulators, inhibitors, odorants, emollients, plasticizer and others.

The term "anionic" refers to a polymeric composite generating in aqueous media a negative electrochemical potential as the result of the presence in its structure of some free acid functional groups capable of dissociating into anions.

In an embodiment described in Fig. 5, the production process starts with the synthesis of copolymer (styrene-alt-maleic anhydride) by bulk co-polymerization using an excess of about 60-90% of maleic anhydride relative to styrene, whereas maleic anhydride works also as a solvent (operation 100).

Copolymerization is typically executed in Sigma-type mixer machines named Hermetic machines in order to be able to work at high pressures e.g. not exceeding 10 bar or in vacuum conditions (less than IOmbar) with double mantle as well with arms equipped with a heating - cooling system.

The copolymerization process for the production of a superabsorbent polymer typically uses styrene monomer stabilized with organic compounds that inhibit the process of homopolymerization during storage and transportation. Such inhibitors are for example substances such as : amino derivatives, thiols derivatives, and hydroxyl derivates as for example (2-hydroxypropyl)-ethylene diamine compounds, 4-tert-butylcatechol and others) being preferred 4-tert-butylcatechol in proportion of 0.002- 0.008% to the monomer, more preferably is 0.003-0.007% to the styrene and most preferably 0.004-0.006% to the styrene and the molar fraction of styrene in the reaction mass is 0.05-0.08, preferably 0.1-0.15 and more preferably 0.18-0.21.

The copolymerization may also contain maleic anhydride that is either fresh maleic anhydride MAnh or recovered maleic anhydride MAnh-R that is recycled from a previous batch as schematically showed in Fig. 5. and the amount of fresh maleic anhydride MAnh relative to recovered maleic anhydride MAnh-R is about 10 - 40% (dry basis).

For the copolymerization, further customary agents may be used such as peroxides, azo compounds etc., which form free radicals by thermal decomposition, while the quantity of initiator is 0.05- 0.15 %, preferably 0.07-0.009% and most preferably 0.08-0.12% to a double quantity of styrene adopted for copolymerization.

Next, it may follow the conversion of styrene-alt maleic anhydride copolymer to styrene-alt maleic acid copolymer by hydrolysis with water (process 200) which is done in the same equipment where copolymerization has been made. Conversion of styrene-alt maleic anhydride copolymer to styrene-alt maleic acid copolymer by hydrolysis using a quantity of water higher than the one stoichiometrical required for total hydrolysis (Ws) with an excess which represents 2-3% versus to the stoichiometrical value, preferably in excess of 4-5% to the stoichiometrical water and most preferably 5-10% in excess to the stoichiometrical water.

The total quantity of water necessary to styrene and maleic anhydride copolymer's hydrolysis is typically inserted in the mass of reaction in two steps from which 50% is in the form of 0.005N hydrochloric acid solution and the rest as non-acidulated water.

The acidulated water is typically inserted into the reaction mass in two portions at a mass reaction's temperature that not exceeding 60°C at 15 minutes intervals between each dosage, and the quantity of non-acidulated water is inserted into the mass of reaction also in two portions, from which the first is after 60 minutes from the last portion of acidulated water, then is inserted the last portion of non-acidulated water and mixing of reaction mass for 45-60 minutes in cooling conditions of 35-40°C. Reaction mass resulted after hydrolysis is a wet solid in form of a powdery mass of white colour with a value of bulk density of 0.6-0.8 g/cm3. Further, mass of reaction that resulted after copolymerization and hydrolysis (which is a blend of styrene maleic acid copolymer - SMAC and free maleic acid -MAC, which contains traces amounts of non-reacted styrene and stabilizer for styrene as well as traces of hydrochloric acid) is transferred to perform the purification process of styrene maleic acid copolymer (process 220).

The purification process which represents the extraction of free maleic acid fraction from SMAC polymer mass is typically done in an equipment such as tank-type with mixing in which over reaction mass resulted after hydrolysis of synthetic copolymer is added a quantity of deionized water for purification [Wp] that represents a quantity correlated to the wet mass of reactions (RM) in accordance with the relationship Wp= 2 * RM or Wp= 5 * RM, preferably Wp= 3 * RM.

Purification generally consists of 3-5 extraction stages followed each time by filtration. The number of extraction and filtration operations are established so that the content of the free carboxylic groups found in polymer of SMAC to be between 0.00909-0.0095 mole/gram, preferably between 0.0091-0.0094 mole/gram and most preferably between 0.0092- 0.0093 mole/gram.

The extraction in fact preferably occurs at temperature of 60°C for 30 minutes and each filtration (process 230) is done with known equipment as press filter or Nuce filter. All solutions resulted from filtering are collected into a tank of supernatant in order to process the maleic acid which it contain.

The processing of maleic acid water solution to recovery of maleic anhydride preferably consists of the following operations: a) concentration of maleic acid solution through reverse osmosis; b) spray drying of maleic acid concentrated solution when resulted maleic acid powder and water; c) conversion of maleic acid powder to maleic anhydride by thermal-vacuum dehydration (with technological parameters modified than those mentioned in US Pat No.4,414,398 when in the end is obtained a material called recovered maleic anhydride (MAnh-R). The purified filtrate of SMAC polymer is typically collected in an equipment as Sigma mixer type in order to be processed with biopolymers to obtain the water soluble composite polymer containing synthetic SMAC polymer and biopolymer [WSPC] (process 300).

The preparation process of polymeric composite [WSPC] containing SMAC and a biopolymer of animal or vegetal origin is preferably preceded by other operations as follows: a) Preparation of a base solution is obtained by dissolution of a solid hydroxide compound in water to 40% by weight, whereas examples of preferred base hydroxide compounds are sodium hydroxide, lithium hydroxide, potassium hydroxide, ammonium hydroxide, preferably sodium hydroxide; b) transformation of styrene maleic acid copolymer obtained in step 230 into styrene-maleic acid monovalent cation salt by neutralization with base solution prepared in a) above in order to obtain a solution of copolymer salt with concentration higher than 25% preferably higher than 35 % and most preferably higher than 50%. Neutralization of the SMAC copolymer is 48-58%, preferably 50-56% and most preferably 52-54%; c) preparation of the biopolymer (as gelatin, albumin, casein, soy, guar or starch, preferably is gelatin) as water solution of 40% by weight in water; d) preparation of polymeric composite is done by treating the solution of styrene maleic acid salt with biopolymer solution at temperature of 55-75°C for 30 minutes.

The amount of biopolymer relative to copolymer in the composite is preferably from 4-6 % (dry basis), preferably 8-10 % (dry basis) and most preferably 12-14 % (dry basis).

The blending of the composite mass typically continues during 4-5 hours until the polymeric mass is transformed from the viscous solution into a partially dried granular mass with a moisture content not higher than 20%. This partially dried polymeric composite material WSPC in granular form is typically subjected to a supplementary drying (process 320) at temperatures of preferably 75-85°C on conveyor belt type or Rotary type in order to obtain final drying of until the moisture content is less than 14%, preferably less than 12% and most preferably less than 8%.

Further steps of drying, grinding and sieving are preferably carried out (process 330 + process 340) to obtain two types of solid phases called herein large solid phase (LSP) with granulometric distribution higher than 100 microns, preferably of 100 -850 microns that corresponds to be used in the manufacture of diapers and a small solid phase (SSF) with granulometric distribution up to 100 microns. The small solid phase SSF is re-used in the preparation of the next new batch of SAP that comes into the process 300.

The large solid phase LSP may be exposed to post-treatment surface coating processes using known chemical substances as: glycerin ethylene glycol, propylene glycol or polyether hydroxyl with properties of biodegradability. Examples of preferred coating materials are hydroxyl polyether, more preferably polyethylene glycol - PEG 200 used at a rate of 0.2-2% by weight (dry basis) to LSP, preferably at a rate of 0.5-1.5% by weight and most preferably at a rate of 0.8-1.2% by weight to LSP.

Surface coating may be applied using equipment like powder coating machines at temperatures of generally 30-70°C, preferably at temperatures of 35-65°C and most preferably at temperatures of 40-60°C for 30 -90 minutes, preferably for 40-75 minutes and most preferably for 50-60 minutes. As a result of this process is resulted the material called Polymer Composite Coated Treated- PCC (operation 350).

The obtained material after surface coating (Polymer Composite Coated) may be subdue to a thermal treatment called first thermal treatment (TT1) (operation 400) consisting in warming of particles mass in hot air with temperature of 90-I40°C for 30-150 minutes, preferably with temperatures of 100-135°C for 45-120 minutes and most preferably by bulk thermal crosslinking at temperatures of 110-120°C, for 60-90 minute using equipment of conveyor belt type or shaking rotary type when is resulted an intermediary material called Polymer Composite Coated first crosslinked (PCC-CL-I).

The material PCC-CL-I may be subdue to a new thermal treatment (TT2) (operation 410) in hot air with temperature of 120-150°C for 5-30 minutes, preferably at temperatures of 125-145°C for 10-25 minutes and most preferably to temperature of 120-140°C for 15-20 minute in the same type of equipment used for TT1 and is obtained the Polymer Composite Coated second crosslinked (PCC-CL-2). The material (PCC-CL-2) may then be conditioned for 24 hours in an atmosphere in which the air has a moisture content of 65% and temperature of 20°C and then is packaged in sealed polyethylene bags (operation 500).

The material resulting after conditioning is a biodegradable SAP with AUL higher than 20g/g in aqueous solution of 0.9% NaCl at pressure of 0.9 psi, which represents the end product of the manufacturing process which is the object of the present invention, i.e. the "High-AUL biodegradable superabsorbent composite polymer" referred to herein.

Reference is made to co-pending application EP21152698.3 for further examples of Low-AUL Bio-SAP and High-AUL Bio-SAPs that may be used in absorbent cores herein, with particular reference to Example 1.

In an embodiment, the first superabsorbent particles (SAP1) are comprised at a level of less than 80%wt, preferably from 10%wt to 32%wt; and the second superabsorbent particles (SAP2) are comprised at a level of at least 20%wt, preferably from 25%wt to 100%wt, more preferably from 30% to 90%, even more preferably from 35% to 80%, even more preferably from 40% to 70%, even more preferably from 45% to 68%, by total weight of superabsorbent particles.

Preferably, the AUL ratio AULSAP1/AULSAP2) of the first superabsorbent particles (SAP1) and second superabsorbent particles (SAP2) is greater than 1.4, preferably greater than 1.5, more preferably from 1.6 to 5, even more preferably from 1.7 to 3. Advantageously, this allows to ensure the right balance of reduced rewet and fast liquid absorption especially on the lower layer and optimal performance under load.

In an embodiment, the first superabsorbent particles (SAP1) have a lower absorption speed (typically according to the vortex method herein) than the second superabsorbent particles (SAP2). Preferably, the vortex time (according to the vortex method herein) of SAP1 is more than 50 seconds, preferably from 60 seconds to 90 seconds. Preferably, the absorption speed of SAP2 is less than 45 seconds, preferably less than 40 seconds, even more preferably from 15 seconds to 35 seconds.

The absorbent inserts herein preferably comprise a wetness indicator (10) which is visible from the exterior of the insert and/or article and which changes appearance when contacted with a body exudates, in particular urine. The wetness indicator (10) may be placed, when seen from the exterior of the insert and/or article, between the two channel-forming areas and/or channels and/or attachment zones according to some embodiments herein.

The wetness indicator serves the role of alerting the wearer that the insert has been soiled and may need changing.

The wetness indicators for use herein may be according to any wetness indicating system known in the art. It is known that wetness indicator can provide an appearing signal, a disappearing signal or a colour change signal, and of course combinations thereof. An appearing signal will typically not be visible or more generally perceivable in the dry insert, and becomes visible or otherwise perceivable when the insert is wet. An appearing signal may for example be provided by a composition which is transparent or having a colour that matches the colour of the backsheet material, which is typically white, in its dry state, and then changes to a different colour when contacted with urine. Other appearing wetness indicator may also be elements capable of providing a physical sensation indicating a fullness level of the absorbent assembly. Examples of such elements are disclosed in WO2008132630 and include a temperature change element (cooling or heating element), a pressure-inducing element or a foam-producing element.

The wetness indicator for use herein may also provide a disappearing signal when the insert is wet. A disappearing signal may be provided by a composition that a first colour when dry and which changes to a second colour that matches the general colour of the backsheet or any graphic printed on the backsheet, so that the second colour is less discernible that the first colour on the insert. Such a disappearing signal may be provided for example by a composition comprising a dye that dissolves in urine and thus fades as the insert is wetted.

The wetness indicator may advantageously provide a colour change signal, which may be typically obtained by a composition having a first colour when dry and a second colour different form the first colour when wet, both colours being discernible by an external observer considering the insert in a dry and a wet state. The wetness indicator may in particular be a colour change composition comprising a suitable pH indicator or another chemical substance that changes colour when contacted with urine. Such compositions are for example disclosed in WO03/070138A2, WO2010/120705 (Klofta) or US2012/165771 (Ruman). The documents cited previously give several examples of such suitable pH indicator, which for example include bromocresol green, bromocresol purple, bromophenol blue, m-cresol purple, cresol red, chlorophenol red, bromothymol blue, bromopyrogallol red, bromoxylenol blue, acridine, or acridine orange, thymolphthalein, thymol blue, xylenol blue, bromochlorophenol blue and indigo carmine. Bromocresol green for example may be applied in a composition having an acid stabilizer so that the pH indicator appears yellow on a dry insert and turns to a green-blue shade when contacted with urine, the typical pH of urine being around pH 7.

More generally, the wetness indicator compositions of the invention may be as disclosed in WO2010/120705 (Klofta) and comprises a colourant, a matrix and a stabilizer. The colourant has an initial colour state, which is associated with a first state of the wetness indicator composition. Examples of this first colour state include, but are not limited to, colours visible to the human eye, such as, red, blue, green, indigo, violet, yellow, orange, purple, and the like; colours not visible to the human eye, such as, colours visible in the ultra violet (or UV), or infra red (or IR) portion of the electromagnetic spectrum, and the like. The first colour state may be invisible, white, black, translucent or opaque. The colourant(s) also has a final colour state, which is associated with a second state of the wetness indicator composition. Examples of this second colour state include, but are not limited to, colours visible to the human eye, such as, red, blue, green, indigo, violet, yellow, orange, purple, and the like; colours not visible to the human eye, such as, colours visible in the UV, or IR portion of the electromagnetic spectrum, and the like. The second colour state may be invisible, white, black, translucent, opaque, or have a change in intensity or visual distinctiveness, and the like, when compared to the first colour state. The initial colour state of the colourant is different, in some form, to the final colour state. For example, the initial colour state may be a first colour, such as, yellow, while the second colour state may be a different colour, such as blue; or the initial colour state may be a first colour, such as, blue, while the second colour state may be transparent, such as, a colour not visible to the human eye, and only visible in the UV portion of the electromagnetic spectrum. In an optional embodiment of the present invention the wetness indicator composition may comprise two or more colourants. The colourant may be employed in compositions at levels which are effective at indicating the presence of a liquid, and include from about 0.001 % to about 5%, from about 0.005% to about 2%, and from about 0.01% to about 1%, and even from 0.01% to 0.5% by weight of the composition.

The compositions for use as wetness indicators herein may comprise a matrix which acts to hold the colourant in place before, during and after contact with liquid. The matrix of the present invention may be highly resistant to colourant leaching, and may be resistant to premature activation in high humidity environments. Upon contact with liquid, such as urine, menses, blood or the like, the matrix allows sufficient liquid to contact the colourant and effect a change in appearance. The matrix concurrently aids in inhibiting the colourant, in either its initial colour state or final colour state, from leaching out of the matrix into the surrounding environment, such as, the absorbent core of a disposable absorbent insert. When the wetness indicating composition is attached to a substrate, the matrix and consequently the composition, should have sufficient wet and dry cohesion, adhesion, and/or flexibility to remain fully retained on the substrate. In other words, the composition retains sufficient flexibility, cohesion, and adhesion to prevent portions of the composition from separating, such as, portions of the composition chipping off or flaking off from the rest of the composition and/or the substrate. Thus, the matrix aids in not only preserving and inhibiting the leaching of the colourant, but it also aids in maintaining the structural integrity of the wetness indicator composition in both the dry and wet states. Such a matrix may include a first and second binding agents, as disclosed in details in WO2010/120705 and may be employed in wetness indicator compositions at levels which are effective at immobilizing and stabilizing the colourant, including from about 5% to about 95%, from about 10% to about 80%, and from about 25% to about 75%, by weight of the composition.

The first binding agent may be any material which immobilizes the colourant when the colourant is in its initial colour state. There are various materials which may be suitable for use as the first binding agent for the wetness indicating compositions of the present invention. The material selected as the first binding agent will be any material which immobilizes the colourant when in its first colour state. In one embodiment of the present invention, possible first binding agents include, but are not limited to, rosins, rosin esters, polymerized rosins, pentaerythritol rosin esters, styrenated terpenes, polyterpene resins, terpene phenolics, and combinations thereof. The first binding agent may be employed in compositions at levels which are effective at immobilizing and stabilizing the colourant in its first state, including from about 4% to about 90%, from about 10% to about 75%, and from about 20% to about 65%, by weight of the composition.

The second binding agent may be any material which immobilizes the colourant when the colourant is in its final colour state. There are various materials which may be suitable for use as the second binding agent for the wetness indicating compositions of the present invention. The second binding agents may be selected from, but are not limited to those second binding agents disclosed in U.S. Pat. No. 6,904,865 to Klofta. The second binding agent may be selected from the group consisting of quaternary ammonium salt compounds, cationic clay, polyacrylic acid polymers, organic acids, and combinations thereof. Examples of suitable quaternary ammonium compounds include, but are not limited to, dimethyl(2-ethylhexylhydrogenatedtallowalkyl) ammonium methyl sulfate, cocoalkylmethyl[ethoxylated(15)] ammonium chloride, dodecyltrimethyl ammonium chloride, hexadecyltrimethyl ammonium methyl sulfate, octadecyltrimethyl ammonium chloride, dicocoalkyldimethly ammonium chloride, di(hydrogenated tallowalkyl)dimethyl ammonium chloride, and distearyldimethyl ammonium chloride. It should be noted that the counter anion associated with the quaternary compound, or any second binding agent having one or more cationic group, is not specifically limited to chloride. Other anions can also be employed and non-limiting examples include methyl sulfate and nitrite. Similarly, any suitable counter cation, such as, but not limited to, sodium, potassium, calcium, magnesium, zinc, protons, ammonium, substituted ammonium and the like, may be associated with a second binding agent having one or more anionic groups.

Wetness indicators herein may further include a stabilizer, as detailed e.g. in WO2010/120705. It may be desirable to include a stabilizer when the colourant is a pH indicator which is particularly advantageous when the absorbent insert is to be stored under conditions of high humidities and temperatures. The inclusion of a stabilizer within the wetness indicator composition is also especially important for new diaper designs where materials and/or chemicals are present that could potentially prematurely activate the colour change of the colourant within the wetness indicator composition. The stabilizer may be an acidic or a basic stabilizer The inclusion of a stabilizer, while not wishing to be limited by theory, is believed to play a role in stabilizing the colourant against premature changes caused by exposure to humid environments and/or certain components of the diaper, by maintaining a stable pH, such as a low pH environment with an acidic stabilizer, around the colourant even when the system is exposed to high humidities and/or certain components of the diaper. This maintenance of a stable pH environment keeps the colourant, especially when the colourant is a pH indicator, in its initial dry colour state. The stabilizer, when present is typically employed in compositions at levels which are effective at stabilizing the colourant, from about 0.001% to about 30%, from about 0.1% to about 15%, and also from about 1% to about 10%, by weight of the composition.

The colour change composition may further be a hot-melt adhesive, which allows for an easy application of the composition on a substrate component of the insert for example by a slot coating process or printed adhesive coating as disclosed e.g. in US2011274834 (Brown). A hot melt adhesive composition may typically become fluid at a temperature of above 60° C. and solidifies when it touches the substrate on which it is applied as it cools down. Hot-melt adhesives may include one or more polymers to provide cohesive strength (e.g., aliphatic polyolefins such as ethylene-propylene copolymers, polyetheramides, polyetheresters, and combinations thereof; ethylene vinyl acetate copolymers; styrenebutadiene or styrene-isoprene block copolymers; etc.), a resin or analogous material (sometimes called a tackifier) to provide adhesive strength (e.g., hydrocarbons distilled from petroleum distillates; rosins and/or rosin esters; terpenes derived, for example, from wood or citrus, etc.); and optional waxes, plasticizers or other materials to modify viscosity (e.g., mineral oil, polybutene, paraffin oils, ester oils, and the like), and/or other additives including, but not limited to, antioxidants or other stabilizers. The matrix may comprise a first and a second binding agent. The matrix acts to hold the colourant in place before, during and after contact with liquid.

The wetness indicator composition may be applied to a layer of the absorbent insert as described herein using a conventional technique, for example printing, spraying or coating, during the making of the absorbent insert. The layer may advantageously be the inner surface of the backsheet or the outer surface of the bottom side of the core wrap or both. This allows the wetness indicator to be visible from the exterior of the insert by transparency through the backsheet while keeping the wetness indicator composition within the insert. The wetness indicator may in particular be easily applied on a layer such a nonwoven or film by a slot-coating process especially if the composition is can be applied as a hot-melt. The slot-coating process allows applying a well-defined slot or a series of slots extending in the machine direction of the converting line, which is typically parallel to the longitudinal direction of the insert.

In an embodiment, the absorbent core (4) comprises a core wrap enclosing the absorbent material (5) therein, and wherein the wetness indicator (10) is positioned between a garment-facing side of the core wrap and a body-facing surface of the backsheet (3), preferably positioned between a garment-facing side of the bottom/lower core wrap (15) and a body-facing surface of the backsheet (3).

The wetness indicator (10) extends along the longitudinal centerline (y) and may cross the transverse centerline (x), wherein the wetness indicator (10) extends from about the transverse centerline (x) (i.e. from a position proximal to the transverse centerline (x) or a position being on the centeriline (x)) to a first terminal position (11) in a direction towards the first transverse edge (6) and extends from about the transverse centerline (x) to a second terminal position (12) in a direction towards the second transverse edge (7); wherein the distance between the first terminal position (11) and the first transverse edge (6) is less than the distance between the second terminal position (12) and the second transverse edge (7) or vice versa. Advantageously this allows for accurate location/fitting of the insert when a specific front-to-back relationship is required. Indeed the fact that the wetness indicator is visually asymmetrically oriented about the transverse centerline allows for creating indicia for the user on the correct front/back positioning to place onto the corresponding outer shell, especially when the outer shell comprises a corresponding window and/or sensor having substantially the same shape of the wetness indicator which need to be overlayed one on top of the other.

Preferably, the wetness indicator (10) has a first maximum width (w) and the window (23) has a second maximum width (W), generally extending along a direction substantially parallel to the transverse centerline (x), and wherein the first maximum width (w) is less than the second maximum width (W). Advantageously this allows for the full wetness indicator (10) to be viewable from the garment facing side of the outer shell (20) when connected thereto.

Preferably the wetness indicator (10) has a first maximum width (w) being greater than 3mm, preferably from 5mm to 30mm, even more preferably from 10mm to 20mm. Advantageously a wider than normal wetness indicator is advantageous for increased ease of viewing through the outer shell (and/or detection by the optical sensor as described herein), however higher widths do not provide significant improvement in visibility and rather add material and cost.

In an embodiment, the absorbent core (4) comprises one or more attachment zones wherein a top layer of a core wrap (14) is adhered to a lower layer of a core wrap (15) such that one or more channels (13) substantially free of absorbent material are formed, preferably wherein the wetness indicator (10) extends between said channels (13) and preferably overlaps at least a portion of said channels. The channels (13) may extend from 10% to 85%, preferably from 15% to 75%, of a length of the core (4) extending in a direction substantially parallel to the longitudinal axis (y).

In a preferred embodiment, channels (13) herein are longitudinally extending and cross both the transverse centerline (x) at a first crossing point and the longitudinal centerline (y) at a second crossing point, wherein the first and second crossing points are different and preferably wherein the second crossing point is positioned closer to the second transverse edge (7) than the first crossing point, and preferably wherein the second transverse edge (7) is positioned proximal to the back region (B) of the outer shell (20) when said insert (1) is connected thereto. Advantageously this allows for improved cup formation in a hybrid concept as well as retaining liquid distribution advantages by guiding exudates to the back of the core.

## THE ABSORBENT ASSEMBLY

Absorbent assemblies herein may comprise an insert (1) as described herein coupled to an outer shell (20) as described herein.

There may be provided a kit of parts comprising: a plurality of inserts (1) as described herein; and one or more outer shells (20) as described herein. Such kits may allow for extended use of the re-usable outer shells.

Preferably, the plurality of inserts (1) differ in at least one of: size, absorbency, biodegradable and/or compostable content, shape, and number of components contained therein. Advantageously this allows for different inserts targeting different types of usage e.g. day vs night, size increases of subjects/wearers etc. whilst retaining substantially the same outer shell.

Preferably, the kits herein further comprising a detection device comprising an optical sensor (OS). Advantageously such allowing the use of one detection device for a plurality of inserts that may be different in at least one of: size, absorbency, biodegradable and/or compostable content, shape, and number of components contained therein as described herein above.

## TEST METHODS

### AUL (Absorbency Under Load, 0.7 psi)

Absorbency Under Load is determined similarly to the absorption under pressure test method No. 442.2-02 recommended by EDANA (European Disposables and Nonwovens Association), except that for each example the actual sample having the particle size distribution reported in the example is measured.

The measuring cell for determining AUL 0.7 psi is a Plexiglas cylinder 60 mm in internal diameter and 50 mm in height. Adhesively attached to its underside is a stainless steel sieve bottom having a mesh size of 36 $\mu$m. The measuring cell further includes a plastic plate having a diameter of 59 mm and a weight which can be placed in the measuring cell together with the plastic plate. The weight of the plastic plate and the weight together weigh 1345 g. AUL 0.7 psi is determined by determining the weight of the empty Plexiglas cylinder and of the plastic plate and recording it as W0. Then 0.900 +/- 0.005 g of water-absorbing polymer or material (particle size distribution 150 - 800 $\mu$m or as specifically reported in the examples which follow) is weighed into the Plexiglas cylinder and distributed very uniformly over the stainless steel sieve bottom. The plastic plate is then carefully placed in the Plexiglas cylinder, the entire unit is weighed and the weight is recorded as Wa. The weight is then placed on the plastic plate in the Plexiglas cylinder. A ceramic filter plate 120 mm in diameter, 10 mm in height and 0 in porosity (Duran, from Schott) is then placed in the middle of the Petri dish 200 mm in diameter and 30 mm in height and sufficient 0.9% by weight sodium chloride solution is introduced for the surface of the liquid to be level with the filter plate surface without the surface of the filter plate being wetted. A round filter paper 90 mm in diameter and < 20 $\mu$m in pore size (S&S 589 Schwarzband from Schleicher & Schüll) is subsequently placed on the ceramic plate. The Plexiglas cylinder holding the material or polymer is then placed with the plastic plate and weight on top of the filter paper and left there for 60 minutes. At the end of this period, the complete unit is taken out of the Petri dish from the filter paper and then the weight is removed from the Plexiglas cylinder. The Plexiglas cylinder holding swollen water-absorbing material or polymer is weighed out together with the plastic plate and the weight is recorded as Wb.

Absorbency under load (AUL) is calculated as follows:

$$AUL0.7psig/g=Wb-Wa/Wa-W0$$

AUL 0.3 psi and 0.5 psi are measured similarly at the appropriate lower pressure.

### Absorption speed (Vortex) measurement:

The vortex test measures the amount of time in seconds required for 2 grams of a superabsorbent material to close a vortex created by stirring 50 milliliters of saline solution at 600 revolutions per minute on a magnetic stir plate. The time it takes for the vortex to close (that the surface of the fluid becomes flat meaning that in the beginning, the centrifugal force that is caused by the rotation of the fluid creates a "coning-in" in the surface, but when the gelling of the SAP proceeds the viscosity of the fluid increases so that the depth of the indentation decreases until it's finally substantially flat) is an indication of the free swell absorbing rate of the superabsorbent material.

Equipment and materials:

1. Beaker, 100 ml.
2. Programmable magnetic stir plate, capable of providing 600 revolutions per minute.
3. Magnetic stir bar without rings, 7.9 mm $\times$ 32 mm, Teflon covered.
4. Stopwatch.
5. Balance, accurate to $\pm$ 0.01 g.
6. Saline solution, 0.9%.
7. Weighing paper.
8. Room with standard condition atmosphere: Temp = 23°C $\pm$ 1°C and Relative Humidity = 50% $\pm$ 2%.

Test procedure:

1. Measure 50 g ± 0.01 g of saline solution into the 100 ml beaker.
2. Place the magnetic stir bar into the beaker.
3. Program the magnetic stir plate to 600 revolutions per minute.
4. Place the beaker on the center of the magnetic stir plate such that the magnetic stir bar is activated. The bottom of the vortex should be near the top of the stir bar.
5. Weigh out 2 g ± 0.01 g of the superabsorbent material to be tested on weighing paper.
6. While the saline solution is being stirred, pour the superabsorbent material to be tested into the saline solution and start the stopwatch. The superabsorbent material to be tested should be added to the saline solution between the center of the vortex and the side of the beaker.
7. Stop the stopwatch when the surface of the saline solution becomes flat, and record the time.
8. The time, recorded in seconds, is reported as the Vortex Time.

It is supposed that the present invention is not restricted to any form of realization described previously and that some modifications can be added to the presented embodiments without reappraisal of the appended claims.

**Claims**

1. A disposable absorbent insert (1) free of transversely extending side panels comprising:

   a liquid permeable topsheet (2);
   a liquid impermeable backsheet (3); and
   an absorbent core (4) comprising absorbent material (5) therein and being sandwiched between said topsheet (2) and backsheet (3);
   wherein the insert (1) comprises a perimeter formed by first and second transverse edges (6, 7) and first and second longitudinal edges (8, 9) connecting the first and second transverse edges (6, 7); a longitudinal centerline (y) extending substantially parallel to said first and second longitudinal edges (8, 9) and interposed therebetween such to divide said insert (1) into a first longitudinal half between said longitudinal centerline (y) and said first longitudinal edge (8) and a second longitudinal half between said longitudinal centerline (y) and said second longitudinal edge (9); and a transverse centerline (x) extending substantially parallel to said first and second transverse edges (6, 7) and interposed therebetween such to divide said insert (1) into a first transverse half between said transverse centerline (x) and said first transverse edge (6) and a second transverse half between said transverse centerline (x) and said second transverse edge (7);

   **characterised in that** the insert (1) comprises a wetness indicator (10) that is viewable from a garment-facing side of the backsheet (3) and **in that** said insert (1) is joinable to a re-usable outer shell (20) and wherein the wetness indicator (10) comprises a composition that changes appearance and/or colour when contacted with urine, the composition comprising a pH indicator and/or a water soluble dye and wherein the absorbent core (4) comprises a core wrap enclosing the absorbent material (5) therein, and wherein the wetness indicator (10) is positioned between a garment-facing side of the core wrap and a body-facing surface of the backsheet (3), and wherein the wetness indicator (10) extends along the longitudinal centerline (y) and from about the transverse centerline (x) to a first terminal position (11) in a direction towards the first transverse edge (6) and extends from about the transverse centerline (x) to a second terminal position (12) in a direction towards the second transverse edge (7); wherein the distance between the first terminal position (11) and the first transverse edge (6) is less than the distance between the second terminal position (12) and the second transverse edge (7) or vice versa.

2. An insert (1) according to any of the preceding Claims wherein the wetness indicator (10) comprises a signal selected from the group consisting of an appearing signal, a disappearing signal, a colour change signal, and a combination thereof.

3. An insert (1) according to any of the preceding Claims wherein the wetness indicator crosses the transverse centerline (x).

4. An insert (1) according to any of the preceding Claims wherein the absorbent core (4) comprises one or more attachment zones wherein a top layer (14) of a core wrap is adhered to a lower layer (15) of a core wrap such that one or more channels (13) substantially free of absorbent material are formed, preferably wherein the wetness indicator (10) extends between said channels.

5. A re-usable outer shell (20) comprising:

   a front (F) and back (B) waist region having uppermost and lowermost edges; and
   a crotch region (C) interposed between the front (F) and back (B) waist region,

wherein the uppermost edges of the waist region form a waist opening and the lowermost edges of the waist region together with lateral extremities of the crotch region form two oppositely disposed leg openings;

and wherein at least the body-facing surface of the crotch region (C) is adapted for receiving an absorbent insert (1) according to any of the preceding claims;

and wherein at least a portion of said crotch region (C) corresponding with the wetness indicator (10) of the insert (1) is translucent and/or transparent such that said wetness indicator (10) is viewable from a garment facing side of said re-usable outer shell (20) or comprises an optical sensor (OS) adapted to automatically detect a colour change of said wetness indicator (10) and send a corresponding signal to an application device.

6. A re-usable outer shell (20) according to Claim 5 comprising re-fastenable or permanent side seams joining the front and back waist regions, said outer shell (20) preferably being in the form of a pant.

7. A re-usable outer shell (20) according to Claim 5 wherein the back waist region (B) comprises, preferably two, transversely extending side panels (21, 21') each comprising a fastener (22, 22') for coupling to a landing zone positioned at a garment facing side of the front waist region (F), said outer shell (20) preferably being in the form of a diaper.

8. A re-usable outer shell (20) according to any of Claims 5 to 7 wherein the crotch region (C) substantially corresponding to the position of the wetness indicator (10), when the insert (1) is joined to the shell (20), comprises: a window (23) for viewing said wetness indicator (10) from a garment-facing side of said shell (20) and/or an optical sensor (OS).

9. A re-usable outer shell (20) according to any of Claims 5 to 8 wherein the optical sensor (OS) is comprised by a detection device further comprising one or more light sources, preferably a plurality of light sources having at least a first colour and a second colour, wherein the first colour is blue and the second colour is red, preferably said light sources consisting of LEDs, more preferably said light sources being positioned at a distance from each other and from the optical sensor; even more preferably the detection device further comprising a battery and a transmitter and being detachably connected to said outer shell.

10. An absorbent assembly comprising:

an insert (1) according to any of Claims 1 to 4;

and

and outer shell (20) according to any of Claims 5 to 9, and preferably further comprising a detection device comprising an optical sensor (OS).

11. A kit of parts comprising:

a plurality of inserts (1) according to any of Claims 1 to 4; and

one or more outer shells (20) according to any of Claims 5 to 9.

12. A kit according to Claim 11 wherein the plurality of inserts (1) differ in at least one of: size, absorbency, biodegradable and/or compostable content, shape, and number of components contained therein.

13. A kit according to any of Claims 11 to 12 further comprising a detection device comprising an optical sensor (OS).

**Patentansprüche**

1. Wegwerfbarer absorbierender Einsatz (1) ohne eine sich quer erstreckende Seitenverkleidung, umfassend:

eine flüssigkeitsdurchlässige obere Folie (2); eine flüssigkeitsundurchlässige hintere Folie (3);und

einen absorbierenden Kern (4), umfassend absorbierendes Material (5) darin und eingezwängt zwischen der oberen Folie (2) und der hinteren Folie (3);

wobei der Einsatz (1) einen Umfang umfasst, der durch erste und zweite quer gerichtete Kanten (6, 7) und erste und zweite längs gerichtete Kanten (8, 9) gebildet ist, die die ersten und zweiten quer gerichteten Kanten (6, 7) miteinander verbindet; eine längs gerichtete Mittellinie (y), die sich im Wesentlichen parallel zu den ersten und zweiten längs gerichteten Kanten (8, 9) erstreckt und dazwischen angeordnet ist, um den Einsatz (1) in eine erste längs gerichtete Hälfte zwischen der längs gerichteten Mittellinie (y) und ersten längs gerichteten Kante (8) und eine zweite längs gerichteten Hälfte zwischen der längs gerichteten Mittellinie (y) und der zweiten längs gerichteten Kante (9) zu trennen; und eine quer gerichtete Mittellinie (x), die sich im Wesentlichen parallel zu der ersten und zweiten quer gerichteten Kante (6, 7) erstreckt und dazwischen derart angeordnet ist, um den Einsatz (1) in eine erste quer gerichtete Hälfte zwischen der quer gerichteten Mittellinie (x) und der ersten quer gerichteten Kante (6) und eine zweite quer gerichteten Hälfte zwischen der quer gerichteten Mittellinie (x) und der zweiten quer gerichteten Kante (7) zu trennen;
**dadurch gekennzeichnet, dass** der Einsatz (1) einen Nässeanzeiger (10) umfasst, der von einer auf die Kleidung gerichteten Seite der hinteren Folie (3) sichtbar ist, und dadurch, dass der Einsatz (1) mit einer wiederverwendbaren äußeren Schale (20) verbunden werden kann, und wobei der Nässeanzeiger (10) eine Zusammensetzung aufweist, die das Aussehen und/oder die Farbe ändert, wenn sie mit Urin in Kontakt kommt, da die Zusammensetzung einen pH-Indikator und/oder einen wasserlöslichen Farbstoff umfasst, und wobei der absorbierende Kern (4) eine Kernumwicklung umfasst, die das absorbierende Material (5) darin einschließt, und wobei der Nässeanzeiger (10) zwischen einer auf die Kleidung gerichteten Seite der Kernumwicklung und einer auf den Körper gerichteten Fläche der hinteren Folie (3) positioniert ist, und wobei sich der Nässeanzeiger (10) entlang der längs gerichteten Mittellinie (y) und von ungefähr der quer gerichteten Mittellinie (x) zu einer ersten Endposition (11) in einer Richtung hin zur ersten quer gerichteten Kante (6) erstreckt und sich von ungefähr der quer gerichteten Mittellinie (x) zu einer zweiten Endposition (12) in einer Richtung hin zur zweiten quer gerichteten Kante (7) erstreckt; wobei der Abstand zwischen der ersten Endposition (11) und der ersten quer gerichteten Kante (6) kleiner ist als der Abstand zwischen der zweiten Endposition (12) und der zweiten quer gerichteten Kante (7) oder umgekehrt.

2. Einsatz (1) nach einem der vorhergehenden Ansprüche, wobei der Nässeanzeiger (10) ein Signal umfasst, ausgewählt aus der Gruppe, bestehend aus einem erscheinenden Signal, einem verschwindenden Signal, einem Farbänderungssignal und einer Kombination davon.

3. Einsatz (1) nach einem der vorhergehenden Ansprüche, wobei der Nässeanzeiger die quer gerichtete Mittellinie (x) kreuzt.

4. Einsatz (1) nach einem der vorhergehenden Ansprüche, wobei der absorbierende Kern (4) eine oder mehrere Befestigungszonen umfasst, wobei eine obere Schicht (14) einer Kernumwicklung an eine untere Schicht (15) einer Kernumwicklung gehaftet ist, so dass ein oder mehrere Kanäle (13) im Wesentlichen ohne absorbierendes Material gebildet sind, vorzugsweise wobei sich der Nässeanzeiger (10) zwischen den Kanälen erstreckt.

5. Wiederverwendbare äußere Schale (20), umfassend:

eine vordere (F) und hintere (B) Hüftregion, die oberste und unterste Kanten aufweist; und eine Schrittregion (C), die zwischen der vorderen (F) und hinteren (B) Hüftregion angeordnet ist, wobei die obersten Kanten der Hüftregion eine Hüftöffnung bilden und die untersten Kanten der Hüftregion zusammen mit den seitlichen Enden der Schrittregion zwei einander gegenüber angeordnete Beinöffnungen bilden; und wobei mindestens die auf den Körper gerichtete Fläche der Schrittregion (C) ausgelegt ist, um einen absorbierenden Einsatz (1) gemäß einem der vorhergehenden Ansprüche aufzunehmen; und wobei mindestens ein Abschnitt der Schrittregion (C), der dem Nässeanzeiger (10) des Einsatzes (1) entspricht, lichtdurchlässig und/oder transparent ist, so dass der Nässeanzeiger (10) von einer auf die Kleidung gerichteten Seite der wiederverwendbaren äußeren Schale (20) sichtbar ist oder einen optischen Sensor (OS) umfasst, der ausgelegt ist, um automatisch eine Farbänderung des Nässeanzeigers (10) nachzuweisen und ein entsprechendes Signal an eine Anwendungsvorrichtung zu senden.

6. Wiederverwendbare äußere Schale (20) nach Anspruch 5, umfassend wiederbefestigbare oder permanente seitliche Nähte, die die vordere und hintere Hüftregion miteinander verbinden, wobei die äußere Schale (20) vorzugsweise die Form einer Hose aufweist.

7. Wiederverwendbare äußere Schale (20) nach Anspruch 5, wobei die hintere Hüftregion (B) vorzugsweise zwei sich quer erstreckende Seitenverkleidungen (21, 21') umfasst, die jeweils eine Befestigung (22, 22') umfassen, um an eine Absatzzone zu koppeln, die an einer auf die Kleidung gerichteten Seite der vorderen Hüftregion (F) positioniert ist, wobei die äußere Schale (20) vorzugsweise die Form einer Windel aufweist.

8. Wiederverwendbare äußere Schale (20) nach einem der Ansprüche 5 bis 7, wobei die Schrittregion (C), die im Wesentlichen der Position des Nässeanzeigers (10) entspricht, wenn der Einsatz (1) mit der Schale (20) verbunden ist, Folgendes umfasst: ein Fenster (23), um den Nässeanzeiger (10) von einer auf die Kleidung gerichteten Seite der Schale (20) zu sehen, und/oder einen optischen Sensor (OS).

9. Wiederverwendbare äußere Schale (20) nach einem der Ansprüche 5 bis 8, wobei der optische Sensor (OS) in einer Nachweisvorrichtung enthalten ist, weiter umfassend eine oder mehrere Lichtquellen, vorzugsweise eine Vielzahl von Lichtquellen, die mindestens eine erste Farbe und eine zweite Farbe aufweisen, wobei die erste Farbe Blau ist und die zweite Farbe Rot ist, vorzugsweise wobei die Lichtquellen aus LEDs bestehen, insbesondere wobei die Lichtquellen in einem Abstand voneinander und vom optischen Sensor positioniert sind; noch bevorzugter wobei die Nachweisvorrichtung weiter eine Batterie und einen Sender umfasst und abnehmbar mit der äußeren Schale verbunden ist.

10. Absorbierende Einheit, umfassend:

    einen Einsatz (1) nach einem der Ansprüche 1 bis 4; und
    eine äußere Schale (20) nach einem der Ansprüche 5 bis 9, und vorzugsweise weiter umfassend eine Nachweisvorrichtung, oder einen optischen Sensor (OS).

11. Kit von Teilen, umfassend:

    eine Vielzahl von Einsätzen (1) nach einem der Ansprüche 1 bis 4; und
    eine oder mehrere äußere Schalen (20) nach einem der Ansprüche 5 bis 9.

12. Kit nach Anspruch 11, wobei sich die Vielzahl von Einsätzen (1) in mindestens einem der Folgenden unterscheidet: der Größe, dem Absorptionsvermögen, dem Gehalt an biologisch abbaubaren und/oder kompostierbaren Stoffen, der Form und der Anzahl der darin enthaltenen Komponenten.

13. Kit nach einem der einem der Ansprüche 11 bis 12, weiter umfassend: eine Nachweisvorrichtung, umfassend einen optischen Sensor (OS).

## Revendications

1. Insert absorbant jetable (1) exempt de panneaux latéraux s'étendant transversalement comprenant :

    une feuille supérieure perméable aux liquides (2) ;
    une feuille arrière imperméable aux liquides (3) ; et
    un noyau absorbant (4) comprenant un matériau absorbant (5) à l'intérieur et étant pris en sandwich entre lesdites feuille supérieure (2) et feuille arrière (3) ;
    dans lequel l'insert (1) comprend un périmètre formé par des premier et second bords transversaux (6, 7) et des premier et second bords longitudinaux (8, 9) reliant les premier et second bords transversaux (6, 7) ; une ligne centrale longitudinale (y) s'étendant sensiblement parallèlement auxdits premier et second bords longitudinaux (8, 9) et interposée entre eux de sorte à diviser ledit insert (1) en une première moitié longitudinale entre ladite ligne centrale longitudinale (y) et ledit premier bord longitudinal (8) et une seconde moitié longitudinale entre ladite ligne centrale longitudinale (y) et ledit second bord longitudinal (9) ; et une ligne centrale transversale (x) s'étendant sensiblement parallèlement auxdits premier et second bords transversaux (6, 7) et interposée entre eux de sorte à diviser ledit insert (1) en une première moitié transversale entre ladite ligne centrale transversale (x) et ledit premier bord transversal (6) et une seconde moitié transversale entre ladite ligne centrale transversale (x) et ledit second bord transversal (7) ;
    **caractérisé en ce que** l'insert (1) comprend un indicateur d'humidité (10) qui est visible depuis un côté faisant face au vêtement de la feuille arrière (3) et **en ce que** ledit insert (1) peut être relié à une enveloppe externe réutilisable (20) et dans lequel l'indicateur d'humidité (10) comprend une composition qui change d'aspect et/ou de couleur lorsqu'elle est en contact avec de l'urine, la composition comprenant un indicateur de pH et/ou un colorant hydrosoluble et dans lequel le noyau absorbant (4) comprend une enveloppe de noyau enfermant le matériau absorbant (5) à l'intérieur, et dans lequel l'indicateur d'humidité (10) est positionné entre un côté faisant face à un vêtement de l'enveloppe de noyau et une surface faisant face au corps de la feuille arrière (3), et dans lequel l'indicateur d'humidité (10) s'étend le long de la ligne centrale longitudinale (y) et d'environ la ligne centrale transversale (x) à une première position terminale (11) dans une direction vers le premier bord transversal (6) et s'étend d'environ la ligne centrale transversale (x) à une seconde position terminale (12) dans une direction vers le second bord transversal (7) ; dans lequel la distance entre la première position terminale (11) et le premier bord transversal (6) est inférieure à la distance entre la seconde position terminale (12) et le second bord transversal (7) ou vice versa.

2. Insert (1) selon l'une quelconque des revendications précédentes dans lequel l'indicateur d'humidité (10) comprend un signal sélectionné dans le groupe consistant en un signal d'apparition, un signal de disparition, un signal de changement de couleur et une combinaison de ceux-ci.

3. Insert (1) selon l'une quelconque des revendications précédentes dans lequel l'indicateur d'humidité croise la ligne centrale transversale (x).

**4.** Insert (1) selon l'une quelconque des revendications précédentes dans lequel le noyau absorbant (4) comprend une ou plusieurs zones de fixation dans lequel une couche supérieure (14) d'une enveloppe de noyau est collée à une couche inférieure (15) d'une enveloppe de noyau de telle sorte qu'un ou plusieurs canaux (13) sensiblement exempts de matériau absorbant sont formés, de préférence dans lequel l'indicateur d'humidité (10) s'étend entre lesdits canaux.

**5.** Enveloppe externe réutilisable (20) comprenant :

une région de taille avant (F) et arrière (B) ayant des bords les plus hauts et les plus bas ; et une région d'entrejambe (C) interposée entre la région de taille avant (F) et arrière (B), dans laquelle les bords les plus hauts de la région de taille forment une ouverture de taille et les bords les plus bas de la région de taille forment avec les extrémités latérales de la région d'entrejambe deux ouvertures de jambe disposées à l'opposé ;
et dans laquelle au moins la surface faisant face au corps de la région d'entrejambe (C) est adaptée pour recevoir un insert absorbant (1) selon l'une quelconque des revendications précédentes ;
et dans laquelle au moins une partie de ladite région d'entrejambe (C) correspondant à l'indicateur d'humidité (10) de l'insert (1) est translucide et/ou transparente de telle sorte que ledit indicateur d'humidité (10) est visible depuis un côté faisant face à un vêtement de ladite enveloppe externe réutilisable (20) ou comprend un capteur optique (OS) adapté pour détecter automatiquement un changement de couleur dudit indicateur d'humidité (10) et envoyer un signal correspondant à un dispositif d'application.

**6.** Enveloppe externe réutilisable (20) selon la revendication 5 comprenant des jonctions repositionnables ou permanentes reliant les régions de taille avant et arrière, ladite enveloppe externe (20) se présentant de préférence sous la forme d'une culotte.

**7.** Enveloppe externe réutilisable (20) selon la revendication 5 dans laquelle la région de taille arrière (B) comprend, de préférence deux panneaux latéraux s'étendant transversalement (21, 21') comprenant chacun une attache (22, 22') pour coupler une zone de réception positionnée au niveau d'un côté faisant face à un vêtement de la région de taille avant (F), ladite enveloppe externe (20) se présentant de préférence sous la forme d'une couche.

**8.** Enveloppe externe réutilisable (20) selon l'une quelconque des revendications 5 à 7 dans laquelle la région d'entrejambe (C) correspondant sensiblement à la position de l'indicateur d'humidité (10), lorsque l'insert (1) est relié à l'enveloppe (20), comprend : une fenêtre (23) pour voir ledit indicateur d'humidité (10) depuis un côté faisant face à un vêtement de ladite enveloppe (20) et/ou un capteur optique (OS).

**9.** Enveloppe externe réutilisable (20) selon l'une quelconque des revendications 5 à 8 dans laquelle le capteur optique (OS) est constitué d'un dispositif de détection comprenant en en outre une ou plusieurs sources lumineuses, de préférence une pluralité de sources lumineuses ayant au moins une première couleur et une seconde couleur, dans laquelle la première couleur est bleue et la seconde couleur est rouge, de préférence lesdites sources lumineuses consistant en DEL, plus préférablement lesdites sources lumineuses étant positionnées à distance les unes des autres et du capteur optique ; plus préférablement encore le dispositif de détection comprenant en outre une batterie et un émetteur et étant relié de manière détachable à ladite enveloppe externe.

**10.** Ensemble absorbant comprenant :

un insert (1) selon l'une quelconque des revendications 1 à 4 ; et
une enveloppe externe (20) selon l'une quelconque des revendications 5 à 9, et de préférence comprenant en outre un dispositif de détection comprenant un capteur optique (OS).

**11.** Kit de pièces comprenant :

une pluralité d'inserts (1) selon l'une quelconque des revendications 1 à 4 ; et
une ou plusieurs enveloppes externes (20) selon l'une quelconque des revendications 5 à 9.

**12.** Kit selon la revendication 11 dans lequel la pluralité d'inserts (1) diffèrent sur au moins l'un parmi : taille, pouvoir absorbant, contenu biodégradable et/ou compostable, forme, et nombre de composants contenus à l'intérieur.

**13.** Kit selon l'une quelconque des revendications 11 à 12 comprenant en outre un dispositif de détection comprenant un capteur optique (OS).

**FIG. 1A**

**FIG. 1B**

1

ADL

2

4

14

5

15

10

3

**FIG. 2**

FIG. 3

20

OS

FIG. 4

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3842017 A1
- EP 3659563 A1
- EP 3895673 A1
- EP 3888606 A1
- US 20200054782 A1
- US 4414398 A
- EP 21152698

- WO 2008132630 A
- WO 03070138 A2
- WO 2010120705 A, Klofta
- US 2012165771 A, Ruman
- US 6904865 B, Klofta
- US 2011274834 A, Brown